# EUROPEAN PATENT APPLICATION

(11) **EP 2 955 220 A1**
(43) Date of publication of application: **16.12.2015**
(21) Application number: 14305888.1
(22) Date of filing: 12.06.2014
(51) Int. Cl.: C12M 1/00, C12M 3/00, C12M 1/42, C12M 1/32, G01N 33/50, C12M 1/12, C12M 3/06, B01L 3/00

(54) **Device for cultivating cells**

(71) Applicant: UNIVERSITE PARIS DESCARTES, 75006 Paris (FR); CENTRE NATIONAL DE LA RECHERCHE SCIENTIFIQUE -CNRS-, 75016 Paris (FR); INSTITUT NATIONAL DE LA SANTE ET DE LA RECHERCHE MEDICALE (INSERM), 75013 Paris (FR); Laboratoire Mabio International, 59200 Tourcoing (FR); UNIVERSITE PARIS-SUD 11, 91400 Orsay (FR)
(72) Inventor: Couraud, Olivier, 78610 AUFFARGIS (FR); Ganeshamoorthy, Kayathiri, 93160 NOISY LE GRAND (FR); Lachman, Vincent, 45430 CHECY (FR); Barik, Samir, 59200 TOURCOING (FR); Uzan, Georges, 94000 VITRY SUR SEINE (FR); Boyer, Julie, 92330 SCEAUX (FR)
(74) Representative: Regimbeau

(57) **Abstract**

The invention related to a device for cultivating cells comprising:
- a lower part comprising at least two lower compartments, a lower wall separating the two lower compartments, and a semi-porous membrane covering the lower compartments, the semi-porous membrane comprising an upper surface intended to receive the cells;
- a first upper part and a second upper part intended to be fitted onto the lower part, in order to obtain selectively:
- a first configuration of the device wherein the first upper part is fitted onto the lower part, the first upper part forming an upper compartment wherein a culture medium can flow, in order to apply shear stress to the cells;
- a second configuration of the device wherein the second upper part is fitted onto the lower part, the second upper part comprising at least two upper compartments, a wall separating the two upper compartments, and injection openings for injecting a substance to be tested in each upper compartment, the wall of the second upper part facing the wall of the lower element and being in contact with the semi-porous membrane so as to prevent a leakage of the injected substance from an upper compartment to another.

## Description

### FIELD OF THE INVENTION

The invention relates to a device for cultivating cells under shear stress. More specifically, the invention relates to a device for cultivating cells and for performing permeability assays.

### BACKGROUND OF THE INVENTION

Many cells in the human body are constantly subjected to fluid shear stresses. Endothelial cells, for example, line the inner surface of blood vessels and are exposed to fluid flow. In particular, the brain endothelial cells form the blood-brain barrier (BBB), a distinctive tissue structure that separates the peripheral blood from the central nervous system. These endothelial cells control the permeability of the BBB, regulating the selective transport from the blood to the brain and vice versa. These endothelial cells are therefore crucial in preventing the entrance of toxic substances to the brain. Interaction with other cells of the neurovascular unit, such as astrocytes, enhances barrier tightness. Due to this tight barrier, the majority of drugs fail to cross the BBB.

Nowadays, most BBB experiments are performed in static Transwell® system. Such a Transwell® system usually comprises an upper chamber and a lower chamber communicating together via a semi-porous membrane with an upper surface receiving the endothelial cells, and filled with culture medium. Such a Transwell® system is configured for performing permeability assays. To this end, a substance is injected into the upper chamber in order to estimate the amount of substance which has been passed through the endothelial cells and the semi-porous membrane and therefore analyze the permeability of the cells to the substance. Such permeability assays are essential for screening for drug candidates.

However, in static Transwell® system, specialized endothelial cells may not fully differentiate, therefore affecting the results of the permeability assays. Indeed, it is well established that for a realistic BBB model, the addition of shear stress to mimic the blood flow is essential, insofar as shear stress positively affects endothelial cell physiology and tight junction formation. In particular, the differentiation and the specialization of the endothelial cells during development are highly dependent upon the nature and magnitude of fluid shear stress. Commonly used *in vitro* shear stress conditioning of endothelial cells includes steady laminar flow or pulsatile flow.

Several devices for cultivating cells under fluid shear stress have therefore been developed.

The document WO2013/086509 describes an example of such a device comprising a lower part provided with wells configured for receiving cells, and an upper part provided with protrusions of complementary shape with the wells of the lower part, so as to form channels wherein culture medium flows under laminar conditions and applies shear stress to the cells.

Another example of device for cultivating cells under fluid shear stress is described in the document Luca Cucullo et al, Immortalized human brain endothelial cells and flow-based vascular modeling: a marriage of convenience for rational neurovascular studies, Journal of Cerebral Blood Flow & Metabolism (2008) 28, 312-328. The device comprises a plurality of hollow fibers provided with an inner wall configured for receiving the cells, and wherein culture medium flows under laminar conditions outside and inside the fibers, so as to apply shear stress to the cells.

The document Balabhaskar Prabhakarpandian et al, SyM-BBB: a microfluidic blood brain barrier model, Lab Chip 2013 March 21, 13(6): 1093-101 discloses another example of device for cultivating cells under fluid shear stress. The device comprises an inner chamber and an outer chamber disposed concentrically relative to each other, and separated to each other via a wall comprising microperforations. The outer chamber is configured for receiving cells, and the outer chamber and the inner chamber are supplied with culture medium flowing under laminar conditions.

However, none of these devices is configured for performing permeability assays.

Another example of device for cultivating cells is described in the document L.M. Griep et al, BBB ON CHIP: microfluidic platform to mechanically and biochemically modulate blood-brain barrier function, Biomed Microdevices 2013 February, 15(1): 145-50. The device comprises a lower part including an upper surface provided with a lower groove extending along a first direction, an upper part including a lower surface provided with an upper groove extending along a second direction different to the first direction, and a semi-porous membrane arranged between the lower part and the upper part, at the intersection of the lower and the upper grooves. The upper part further comprises openings leading to the upper groove and the lower groove so that culture medium flows under laminar conditions along the upper and the lower grooves and applies shear stress to the cells. The lower part is further provided with an electrode in order to perform permeability assays of the cells to ions.

However, such a device is not configured for performing permeability assays with substances other than ions. Such a device is therefore not configured for screening for drug candidates. Moreover, such a device is an experimental device which is not adapted to industry purposes.

### SUMMARY OF THE INVENTION

The present invention aims to solve the problems of the prior art devices.

In particular, in a given embodiment, the invention proposes a device for cultivating cells which, in a first configuration, is configured for applying shear stress on cells, and in a second configuration, is configured for performing permeability assays on the cells grown when the device was in the first configuration.

More specifically, in one embodiment, the invention proposes a device for cultivating cells comprising:
- a lower part comprising at least two lower compartments, a lower wall separating the two lower compartments, and a semi-porous membrane covering the lower compartments, the semi-porous membrane comprising an upper surface intended to receive the cells;
- a first upper part and a second upper part intended to be fitted onto the lower part, in order to obtain selectively:
- a first configuration of the device wherein the first upper part is fitted onto the lower part, the first upper part forming an upper compartment wherein a culture medium can flow, in order to apply shear stress to the cells;
- a second configuration of the device wherein the second upper part is fitted onto the lower part, the second upper part comprising at least two upper compartments, a wall separating the two upper compartments, and injection openings for injecting a substance to be tested in each upper compartment, the wall of the second upper part facing the wall of the lower element and being in contact with the semi-porous membrane so as to prevent a leakage of the injected substance from an upper compartment to another.

Preferably, each upper part comprises an upper channel and the lower part comprises a lower channel leading to the lower compartments, the upper channels being arranged so that when the upper part is fitted onto the lower part, each upper channel is connected to a lower channel.

Preferably, the upper channels and the lower channels are arranged such that each lower compartment communicates with at least two upper channels via the lower channels.

Preferably, at least some of the upper channels are filled with silicon stoppers.

Preferably, the lower part and the upper parts are made of a transparent material.

Preferably, the wall separating the lower compartments is thicker than the wall separating the upper compartments of the second upper element.

Preferably, the device further comprises at least one electrode arranged in at least one lower compartment.

### BRIEF DESCRIPTION OF THE DRAWINGS

The above, and other objects, features and advantages of this invention, will be apparent in the following detailed description which is to be read in connection with the accompanying drawings wherein:
- Figure 1 is an exploded view of a device for cultivating cells in a first configuration according to one embodiment of the invention;
- Figure 2 is an exploded view of the device illustrated in Figure 1 in a second configuration;
- Figure 3 is a top view of a lower part of the device illustrated in Figures 1 and 2;
- Figure 4 is a side view of the lower part illustrated in Figure 3;
- Figure 5 is a top view of a support part of the device illustrated in Figures 1 and 2;
- Figure 6 is a bottom view of a first upper part of the device illustrated in Figure 1;
- Figure 7 is a bottom view of the second upper part of the device illustrated in Figure 2;
- Figure 8 shows that flow promotes hCMEC/D3 quiescence;
- Figures 9a and 9b show that flow ameliorates expression of adherent and TJ protein in hCMEC/D3 cells;

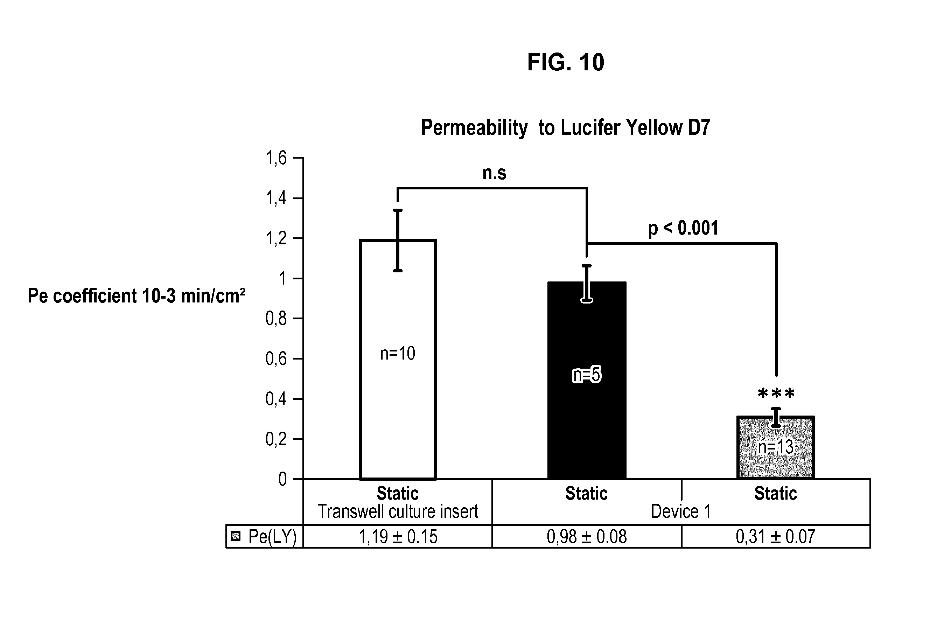

- Figure 10 shows that flow significantly decreases paracellular permeability of Lucifer yellow of hCMEC/D3 cells;

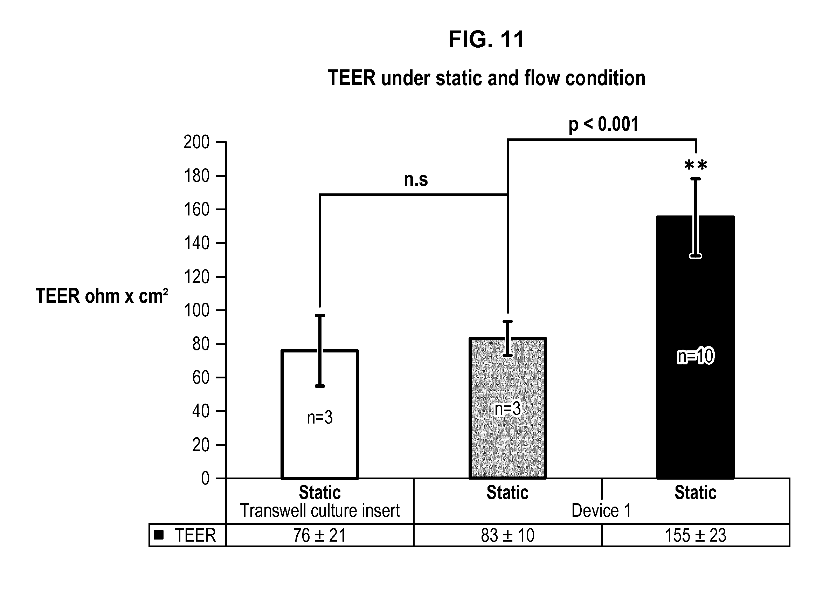

- Figure 11 shows that flow significantly increases transendothelial electrical resistance of hCMEC/D3 cells;
- Figure 12 shows that flow increases Pgp and BCRP activities of hCMEC/D3 cells;
- Figure 13 shows that permeability to drug is significantly modified under flow in hCMEC/D3 cells;
- Figure 14 shows that the cellular orientation of the circulating endothelial progenitor (CEP) cells cultured under shear stress compared to static conditions;
- Figure 15: shows the switch of gene expression from venous to arterial phenotype under flow in CEP cells.

### DETAILED DESCRIPTION OF THE INVENTION

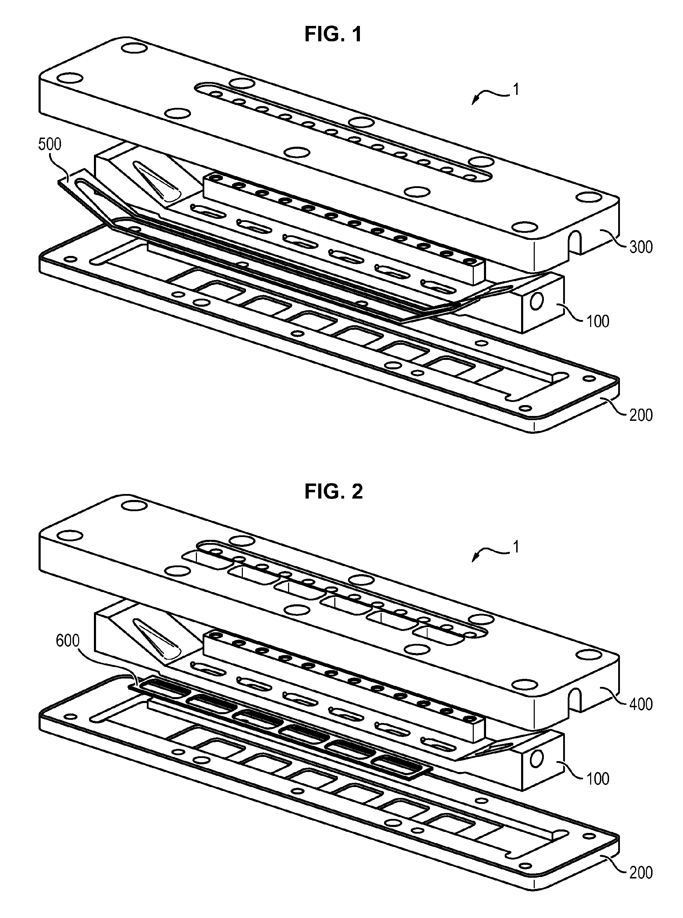

Figures 1 and 2 show a device 1 for cultivating cells, such as endothelial cells.

The device 1 comprises a lower part 100 intended to be fitted onto a support plate 200, as well as a first upper part 300 and a second upper part 400 intended to be fitted onto the lower part 100. In a first configuration of the device 1, the first upper part 300 is fitted into the lower part 100, whereas in a second configuration of the device 1, the second upper part 400 is fitted onto the lower part 100. The first configuration and second configuration are illustrated in Figures 1 and 2, respectively.

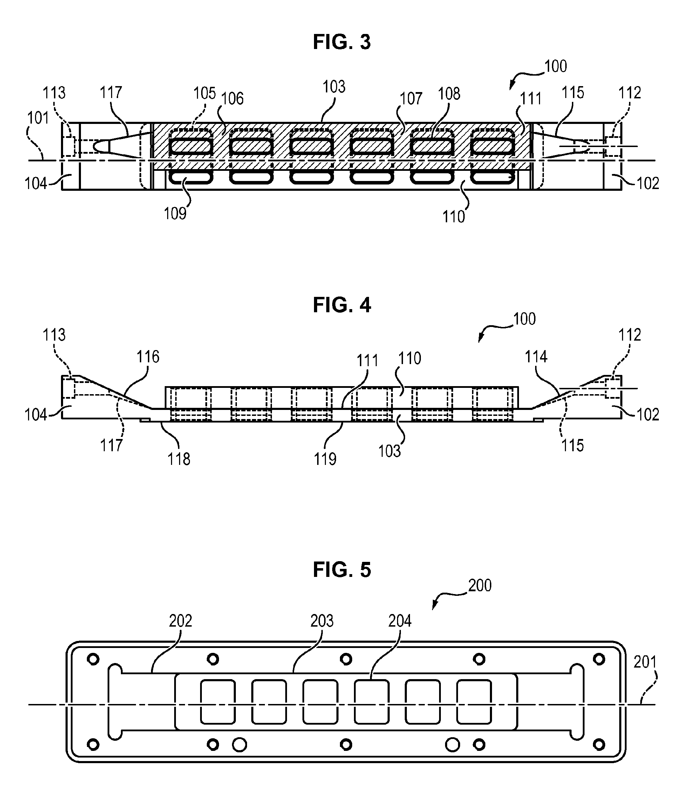

A more detailed view of the lower part 100 is represented in Figures 3 and 4.

The lower part 100 extends along a longitudinal axis 101 and comprises a proximal portion 102, a medium portion 103 and a distal portion 104.

The medium portion 103 of the lower part 100 is provided with at least two lower compartments 105, and a lower wall 106 separating the lower compartments 105. The lower compartments 105 are aligned along the longitudinal axis 101, and the lower wall 106 extends substantially perpendicularly to said longitudinal axis 101.

According to the embodiment illustrated in Figures 3 and 4, the medium portion 103 of the lower part 100 is provided with six lower compartments 105 aligned along the longitudinal axis 101, and each pair of adjacent lower compartments 105 is separated by a lower wall 106 extending substantially perpendicularly to the longitudinal axis 101.

The medium portion 103 of the lower part 100 further comprises a planar upper surface 107 covering the lower compartments 105 and provided with lower openings 108 leading to each lower compartment 105, so that lower openings 108 provide access to the lower compartments 105. The lower openings 108 are aligned longitudinally and are shifted transversally on a first side of lower part 100 with respect to the longitudinal axis 101.

The medium portion 103 of the lower part 100 also includes lower channels 109 leading to the lower compartments 105, so that the lower channels 109 provide access to the lower compartments 105. A lower channel 109 is for example associated to each lower compartment 105. According to the embodiment illustrated in Figures 3 and 4, the lower channels 109 are provided in a longitudinal wall 110 extending substantially perpendicularly from the upper surface 107. The wall 110 is shifted transversally on a second side of the lower part 100 with respect to the longitudinal axis 101.

The medium portion 103 of the lower part 100 comprises a semi-porous membrane 111 covering the lower compartments 105 and including an upper surface intended to receive the cells. According to the embodiment illustrated in Figures 3 and 4, the semi-porous membrane 111 covers the lower openings 107, therefore extending on the first side of the medium portion 103. The semi-porous membrane 111 is for example heat sealed to the upper surface 107 of the medium portion 103. The semi-porous membrane 111 is for example made of polycarbonate and comprises pore size of approximately 0.4µm. in the first configuration of the device 1, the lower compartments 105 are for example maintained full of culture medium to allow nutrients access to the cells through the semi-porous membrane 111.

The proximal portion 102 and the distal portion 104 of the lower part 100 are provided with an inlet 112 and an outlet 113, respectively. The inlet 112 and the outlet 113 are shifted transversally on the first side of the lower part 100 with respect to the longitudinal axis 101 for providing culture medium flow over the upper surface of the semi-porous membrane 111, when the device 1 is in the first configuration. In the first configuration, the inlet 112 and the outlet 113 are for example connected to culture medium reservoir by means of an inlet pipe and an outlet pipe, respectively. The culture medium reservoir comprise for example a compact cell culture cassette CLINIcell® commercialized by MABIO connected in series to a peristaltic pump. The inlet pipe and the outlet pipe are for example inserted and glued into the inlet 112 and the outlet 113, at the ends of the proximal portion 102 and the distal portion 104, respectively.

According to the example illustrated in Figures 3 and 4, the proximal portion 102 of the lower part 100 is beveled so as to form a downward slope 114 leading from the inlet 112 to the upper surface 107 of the medium portion 103. The downward slope 114 includes for example a proximal lower groove 115 extending from the inlet 112 to the upper surface 107 of the medium portion 103 for directing the culture medium flow to the upper surface of the semi-porous membrane 111. The width of the proximal lower groove 115 increases from the inlet 112 to the upper surface 107 of the medium portion 103, while the depth of the proximal lower groove 115 decreases from the inlet 112 to the upper surface 107 of the medium portion 103.

According to the example illustrated in Figures 3 and 4, the distal portion 104 of the lower part 100 is beveled so as to form an upward slope 116 leading from the upper surface 107 of the medium portion 103 to the outlet 113. The upward slope 116 includes a distal lower groove 117 extending from the inlet 112 to the upper surface 107 of the medium portion 103 for directing the culture medium flow to the outlet 113. The width of the distal lower groove 117 decreases from the inlet 112 to the upper surface 107 of the medium portion 103, while the depth of the distal lower groove 117 increases from the inlet 112 to the upper surface 107 of the medium portion 103.

The inlet 112, the outlet 113, the downward slope 114, the upward slope 116, the proximal lower groove 115, and the distal lower groove 117 of the lower part 100 are sized and dimensioned so that the culture medium flows under laminar conditions over the upper surface of the semi-porous membrane 111.

The lower part 100 is preferably made of a transparent material. In this way, the cells can be visualized under the microscope at any moment of the experiment. The lower part 100 is for example made of polystyrene, polyethylene terephthalate, or polycarbonate.

The lower part 100 further gives the opportunity to inject cells, such as perivascular cells, into the lower compartments 105 and therefore perform coculture or triculture assays. These cells are not subjected to shear stress.

A more detailed view of the support plate 200 is represented in Figure 5.

The support plate 200 extends along a longitudinal axis 201. When the lower part 100 is fitted onto the support plate 200, the longitudinal axis 101 of the lower part 100 is substantially parallel to the longitudinal axis 201 of the support plate 200.

The support plate 200 is provided with a groove 202 configured for receiving the lower part 100 so as to block translation movements of the lower part 100 with respect to the plane formed by the support plate 200, when the lower part 100 is fitted onto the support plate 200.

According to the embodiment illustrated in Figure 5, the groove 202 of the support plate 200 includes a recess 203 configured for receiving a corresponding protrusion 118 extending from a lower surface 119 of the medium portion 103 of the lower part 100 opposite to the upper surface 107, so as to block translation movements of the lower part 100 with respect to the plane formed by the support plate 200.

The groove 202 of the support plate 200 further comprises openings 204 intended to face the lower compartments 105, when the lower part 100 is fitted into the support plate 200. In this way, when the lower part 100 is made of transparent material, the user can visualize under the microscope the lower compartments 105 and the cells disposed on the semi-porous membrane 111 at any moment of the experiment.

The support plate 200 is for example made of metal, such as aluminum.

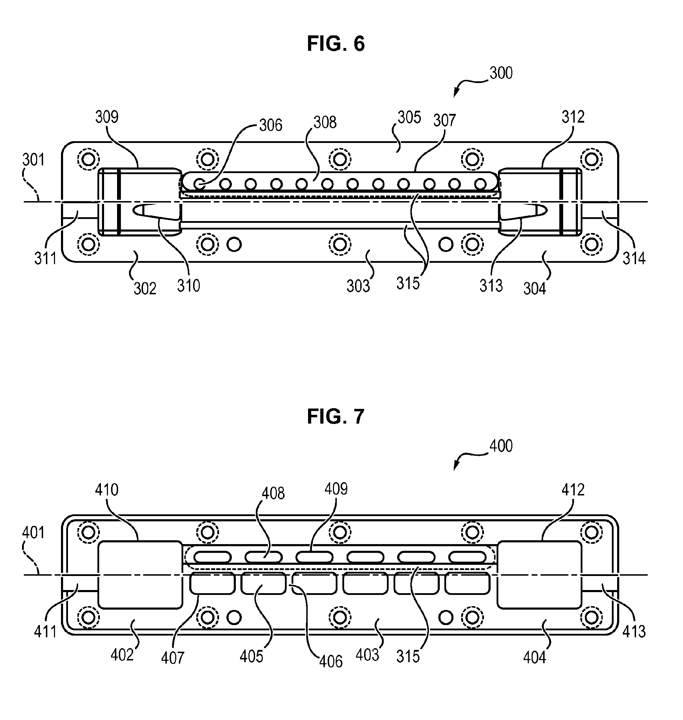

A more detailed view of the first upper part 300 is represented in Figure 6.

The first upper part 300 extends along a longitudinal axis 301 and comprises a proximal portion 302, a medium portion 303 and a distal portion 304. In the first configuration of the device 1, when the first upper part 300 is fitted onto the lower part 100, the longitudinal axis 101 of the lower part 100 is substantially parallel to the longitudinal axis 301 of first upper part 300.

The first upper part 300 can be removably attached to the support plate 200 using screws and corresponding threaded openings provided in the support plate 200 and in the first upper part 300.

The first upper part 300 is dedicated to cell culture under laminar flow conditions. When the device 1 is in the first configuration, the first upper part 300 forms an upper compartment wherein the culture medium can flow, in order to apply shear stress to the cells. In the first configuration of the device 1, the culture medium is caused to flow in the upper compartment from the inlet 112 to the outlet 113 of the lower part 100. To this end, the medium portion 303 of the first upper part 300 is provided with a lower surface 305 forming with the upper surface of the semi-porous membrane 111 the upper compartment, when the first upper part 300 is fitted onto the lower part 100. The upper compartment is sized and dimensioned so that the culture medium flow is laminar over the upper surface of the semi-porous membrane 111.

The medium portion 303 of the first upper part 300 further includes first upper channels 306 arranged in such a way that, when the first upper part 300 is fitted onto the lower part 100, each first upper channel 306 is connected to a lower channel 109, and therefore communicates with the lower compartments 105. The first upper channels 306 and the lower channels 109 allow the user to inject substances into the lower compartments 105 or to draw substances from the lower compartments 105.

Preferably, the first upper channels 306 and lower channels 109 are arranged in such a way that each lower compartment 105 communicates with at least one first upper channels 306 via the lower channels 109, so as to equilibrate the pressure between the lower compartments 105 and the outside, while injecting or drawing substances in the lower compartments 105.

In the example illustrated in Figure 6, the first upper channels 306 are arranged in a first upper opening 307 facing the lower channels 109, when the first upper part 300 is fitted into the lower part 100, and filled with a silicon stopper 308. Silicon protrusions are for example arranged on said silicon stopper for indicating the position of the first upper channels 306. In other words, the silicon protrusions indicate where the needle needs to be inserted to access to the lower compartments 105.

The proximal portion 302 of the first upper part 300 comprises a proximal recess 309 of complementary shape to the corresponding proximal portion 102 of the lower part 100. In particular, the proximal recess 309 is provided with an upward slope extending to the medium portion 303 of the first upper part 300. The proximal recess 309 further comprises a proximal upper groove 310 of similar shape as the proximal lower groove 115. The proximal upper groove 310 is intended to face the proximal lower groove 115, so that, in the first configuration of the device 1, the proximal upper groove 310 and the proximal lower groove 115 form together a channel that directs the culture medium flow from the inlet 112 to the upper surface of the semi-porous membrane 111.

In the example illustrated in Figure 5, the proximal portion 302 of the first upper part 300 further includes an inlet groove 311 extending longitudinally from the proximal recess 309 to the end of the proximal portion 302 of the first upper part 300, and configured for receiving the inlet pipe.

The distal portion 304 of the first upper part 300 comprises a distal recess 312 of complementary shape to the corresponding distal portion 104 of the lower part 100. The distal recess 312 is provided with a downward slope extending from the medium portion 303 of the first upper part 300. The distal recess 309 further comprises a distal upper groove 313 of similar shape as the distal lower groove 117. The distal upper groove 313 is intended to face the distal lower groove 117, so that, in the first configuration of the device 1, the distal upper groove 313 and the distal lower groove 117 form together a channel that directs the culture medium flow from the upper surface of the semi-porous membrane to the outlet 113.

In the example illustrated in Figure 6, the distal portion 304 of the first upper part 300 further includes an outlet groove 314 extending longitudinally from the distal recess 312 to the end of the distal portion 304 of the first upper part 300, and configured for receiving the outlet pipe.

The first upper part 300 is preferably made of a transparent material. In this way, the cells can be visualized under the microscope at any moment of the experiment. The first upper part 300 is for example made of polystyrene, polyethylene terephthalate, or polycarbonate.

In the first configuration, the device 1 is further provided with a first joint 500 surrounding the lower openings 108, the proximal lower groove 115, and the distal lower groove 117 so as to prevent leakage of the culture medium, while flowing over the upper surface of the semi-porous membrane 111. The lower surface 305 of the medium portion 303 of the first upper part 300 includes for example grooves 315 extending longitudinally on both sides of the lower openings 108, when the first upper part 300 is fitted onto the lower part 100, and configured for receiving the first joint 500.

A more detailed view of the second upper part 400 is represented in Figure 7.

The second upper part 400 extends along a longitudinal axis 401 and comprises a proximal portion 402, a medium portion 403 and a distal portion 404. In the second configuration of the device 1, when the second upper part 400 is fitted onto the lower part 100, the longitudinal axis 101 of the lower part 100 is substantially parallel to the longitudinal axis 401 of the second upper part 400.

The second upper part 400 can be removably attached to the support plate 200 using screws and corresponding threaded openings provided in the support plate 200 and in the second upper part 400.

The second upper part 400 is dedicated to perform permeability assays on the cells grown, when the device 1 is in the second configuration.

The medium portion 403 of the second upper part 400 is provided with at least two upper compartments 405, an upper wall 406 separating the two upper compartments 405 and injection openings 407 for injecting a substance to be tested in each upper compartment 405. The upper compartments 405 and the injection openings 407 are aligned longitudinally, and the upper wall 406 extends substantially perpendicularly to the longitudinal axis 401. The upper compartments 405 preferably face the semi-porous membrane 111 and the lower openings 108, when the device 1 is in the second configuration.

According to the embodiment illustrated in Figure 7, the medium portion 403 of the second upper part 400 comprises six upper compartments 405 aligned longitudinally, and each pair of adjacent upper compartments 405 is separated by an upper wall 406 extending substantially perpendicularly to the longitudinal axis 401.

In the second configuration of the device 1, when the second upper part 400 is fitted onto the lower part 100, each upper wall 406 faces a corresponding lower wall 106 and is in contact with the semi-porous membrane 111 so as to prevent leakage of the injected substance from an upper compartment 405 to another. Preferably, the lower walls 106 are thicker than the upper walls 406 of the second upper compartment 405 so that limited damage occurs to the cells arranged in front of the lower openings 108.

The medium portion 403 of the second upper part 400 further includes second upper channels 408 arranged in such a way that, when the second upper part 400 is fitted onto the lower part 100, each second upper channel 408 is connected to a lower channel 109, and therefore communicates with the lower compartments 105. The second upper channels 408 and the lower channels 109 allow the user to inject substances into the lower compartments 105 or to draw substances from the lower compartments 105.

In the example illustrated in Figure 7, each second upper channel 408 forms a second upper opening 409 facing the corresponding lower channel 109.

The proximal portion 402 and the distal portion 404 of the second upper part 400 comprise a proximal recess 410 and a distal recess 412 respectively configured for receiving the proximal portion 102 and the distal portion 104 of the lower part 100.

In the example illustrated in Figure 7, the proximal portion 402 and the distal portion 404 of the second upper part 400 further include an inlet groove 411 and an outlet groove 413 extending longitudinally from the proximal recess 410 and the distal recess 412 to the end of the proximal portion 402 and the distal portion 403, and configured for receiving the inlet pipe and the outlet tube, respectively.

The second upper part 400 is preferably made of a transparent material. In this way, the cells can be visualized under the microscope at any moment of the experiment. The second upper part 400 is for example made of polystyrene, polyethylene terephthalate, or polycarbonate.

In the second configuration, the medium portion 103 of the lower part 100 is further provided with a second joint 600 surrounding the lower openings 108, and separating the lower openings 108 from each other so as to prevent leakage of the injected substances from a second upper compartment 405 to another. In this case, the upper walls 406 are in contact with the semi-porous membrane 111 by means of the second joint 600.

According to another embodiment of the invention (not illustrated), at least one lower compartment 105 is provided with at least one electrode, in order to perform permeability assays to ions. The electrodes are for example inserted into the lower compartments 105 via the second upper channels 406 and the corresponding lower channels 109.

The device 1 is for example used as follows.

The lower part 100 is firstly fitted onto the support plate 200, and the lower compartments 105 are filled with culture medium and nutrients. Then, a monolayer of cells is deposited on the semi-porous membrane 111. Next, the first upper part 300 is fitted onto the lower part 100 and removably attached to the support plate 200. The device 1 is in the first configuration. The inlet pipe and the outlet pipe are connected to the culture medium reservoir and laminar culture medium flow is provided over the upper surface of the semi-porous membrane 111, so as to apply shear stress to the cells. A maximum flow shear stress of 20 dynes/cm² can be applied to the cells.

After the cell growth has been completed, the first upper part 300 is removed from the lower part 100 and the support plate 200, and the second upper part 400 is fitted into the lower part and removably attached to the support plate 200. The device 1 is in the second configuration. Then, the substance to be injected is injected into the upper compartments 405. Next, the culture medium disposed in the lower compartments 105 is drawn in order to estimate the amount of injected substance which has been passed through the monolayer of cells and the semi-porous membrane 111, and therefore analyze the permeability of the cells to the injected substance.

The device 1 described below allows, in the first configuration, when the first upper part 300 is fitted onto the lower part 100, to cultivate cells under flow shear stress, and in the second configuration, when the second upper part 400 is fitted onto the lower part 100, to perform permeability assays on the cells previously grown under flow shear stress.

Such a device 1 is therefore particularly advantageous insofar as the permeability assays can be performed in parallel on cells which have been grown under the same conditions, only by replacing the first upper part 300 by the second upper part 400.

Moreover, such a device 1 provides the advantage that it can be used for cultivating adherent cells, preferably endothelial or epithelial cells, more preferably ependymal cells. Cells cultivated by this method display a phenotype which is closer to that of corresponding cells in situ than any other methods of the art.

In another aspect, the invention thus provides a method for cultivating adherent cells, said method comprising the following steps:
i) injecting culture medium in the lower compartment of a device 1 of the invention;
ii) seeding said device 1 with adherent cells, preferably endothelial or epithelial cells, more preferably ependymal cells;
iii) growing said cells in static conditions until the formation of a cellular monolayer is obtained; and
iv) applying to said cellular monolayer a shear stress comprised between 0.01 dyne/cm² to 30 dynes/cm² until a mature cellular monolayer is obtained for example, preferably during 2 to 4 days, more preferably during 3 days.

The "adherent cells" within the meaning of the invention, are established cell lines or cells obtained directly from the extraction of animal or human tissues healthy or tumor, which, in the culture conditions used, need a solid support to multiply and grow normally. They usually form a single layer on their support due to the phenomenon of contact inhibition. It therefore excludes the cells that do not need a solid support to multiply and grow in suspension in the culture medium. The adherent cell lines can be derived from primary cultures of normal cells or tumor cells but also can be obtained by transforming cells with immortalizing agents as is the case of the line PER.C6.

Adherent cells are well-known in the art and include such cells as e.g. endothelial and epithelial cells, in particular ependymal cells.

The term "epithelial cells", as used herein, refers to cells which constitute tissues consisting of closely juxtaposed cells. Epithelial cells are thus bound together in sheets of tissue called epithelia. These sheets are held together through several types of interactions, including tight junctions, adherens, desmosomes, and gap junctions. Given the wide distribution, epithelial cells perform a variety of functions: protection (skin), absorption (intestine), secretion (glands), excretion (kidney), gas exchange (lung and blood vessels). The junctions between the epithelial cells are particularly important because they restrict free movement of compounds (e.g. ions, proteins, etc.) both within and through the epithelium. In this regard, tight junctions perform a critical function besides cell adhesion. Tight junctions regulate the passage of ions and small metabolites, and restrict the diffusion of molecules between neighboring cells (paracellular). Epithelia comprise such tissues as e.g. epidermis, intestinal epithelium, respiratory epithelium, bladder, and epithelium of uro-genital organs.

One particular type of epithelia is the ependyma, which is the thin lining of cells around the central nervous system, i.e. the brain and the spinal cord. As used herein, the term "ependymal cells" thus refers to any cell residing partly or completely in the ependymal layer in the CNS ventricular system or the same cell type located elsewhere. Ependymal cells are multiciliated epithelial cells that line the walls of the ventricular system in the adult brain. More generally, an "ependymal cell" is defined herein to include any cell having the characteristics of an ependymal cell isolated from the ependymal cell layer of post-natal CNS tissue.

The term "endothelial cells", as used herein, refers to cells that line the interior surface of blood vessels and lymphatic vessels, forming an interface between circulating blood or lymph in the lumen and the rest of the vessel wall. Endothelial cells ensure a semi-selective barrier between the vessel lumen and surrounding tissue, controlling the passage of materials and the transit of white blood cells into and out of the bloodstream. Specifically, in the central nervous system, the endothelial cells are connected by tight junctions and thus participate in the blood-brain barrier. Endothelial cells which can be cultivated by the method of the present invention include, endothelial cells from large and small blood vessels (e.g., microvascular cells). Examples of endothelial cells from large blood vessels include, without limitation, umbilical vein endothelial cells, umbilical artery endothelial cells, pulmonary artery endothelial cells, saphenous vein endothelial cells. Other useful endothelial cells, include, without limitation, lung microvascular cells, coronary artery endothelial cells, cutaneous microvascular endothelial cells, aortic endothelial cells. The endothelial cells may be mature or immature. In one embodiment, the endothelial cells are human.

As an example of adherent cell lines suitable for the purpose of the invention, mention can be made of murine cell lines such as 3T3, NTCT, WEHI, the hamster cell lines such as BHK (including line BHK21) or CHO cell lines such as the line MDCK canine, porcine cell lines such as the line PKI 5 cell lines as bovine MDBK line, simian cell lines such as Vero, LLC-MK2, FRHL2, MA104 the human cell lines such as the line MRC5, 293, PER.C6, HeLa. Further examples of adherent cells which can be used in the methods of the invention are found in the experimental examples of the present disclosure. In particular, brain endothelial cells are the human cell line hCMEC/D3.

The composition of the culture medium filled in the lower part of the device 1 in first step of the method of the invention will depend upon the type of adherent cell which is to be grown; the skilled person will easily determine those based on the general knowledge of said person. Advantageously, non-adherent cells, such as neurosphere, or adherent cells, such as pericytes or astrocytes, can be introduced with culture medium in the lower part of the device 1 in order to fully recreate a physiological environment such as a neurovascular unit environment.

In the second step of the method of the invention, the adherent cells are grown in static conditions until a monolayer of cells is formed. It will be easily conceived that this step corresponds to the usual step of growing adherent cells on a substrate. In the present case, the substrate is provided by the semi-porous membrane 111 of the device of the invention.

In the final step of said method, the cells are submitted to a flow shear stress, as disclosed hereinabove. This shear stress is particularly advantageous since it leads to obtaining a mature cellular monolayer. A "mature cellular monolayer" as used herein is a single layer of cells which present structural and functional characteristics remarkably similar to the ones of the cells in vivo. Indeed, they present a phenotype, e.g. an endothelial, epithelial or ependymal phenotype, closer to that of corresponding cells in situ than any other methods of the art. In particular, those mature cells display improved expression and/or localization of adherens and tight junction proteins, as well as of several general transporters, including the multidrug resistance transporters, as compared to cells grown under static conditions only. This translates into increased tight junction integrity and decreased permeability. This feature is particularly advantageous because it allows the generation of immortal cells which behave in cell culture as regular cells in situ. For instance, the mature cellular monolayer of the hCMEC/D3 cell line used in the examples of the invention displays a phenotype much closer to that of human brain endothelial cells in situ (forming the human blood-brain barrier) than cells described in the prior art. In particular such a mature monolayer of hCMEC/D3 cells exhibits:
- A permeability to Lucifer Yellow inferior to 0.60 10⁻³ cm.min⁻¹, preferably inferior to 0.40 10⁻³ cm.min⁻¹
- A measured TEER superior to 100 ohms.cm², preferably superior to 120 ohms.cm²
- A strong expression and activity of ABC transporters, including P-gp and BCRP (respectively also known as ABCB1 and ABCG2).

In another aspect, the invention thus also relates to a mature cellular monolayer of adherent cells obtained by the method described above.

The device 1 of the invention is highly advantageous in that it can be used for predicting absorption rate of candidate drugs or any test compounds across adherent cell layers, such as the intestinal epithelial membrane or the brain blood barrier. For example, Caco-2 cells are widely used across the pharmaceutical industry as an in vitro model of the human small intestinal mucosa to predict the absorption of orally administered drugs. When cultured as a confluent monolayer on a cell culture insert filter (e.g., Transwell®), the cells differentiate to form a polarized epithelial cell monolayer that provides a physical and biochemical barrier to the passage of ions and small molecules. In this situation, the cell layer allows the study of drug transport from the apical side to the basolateral side (A to B direction) as well as from the basolateral side to the apical side (B to A direction) of a test compound. Apparent permeability (Papp) values of test compounds are calculated and compared to values of reference control compound of known permeability.

However, even a model as widely used as Caco-2 does not reproduce genuinely the behavior of human tissues such as the ependymal, BBB or the intestinal epithelium, and may thus lead to differences in appreciation of the permeability of test compounds.

On the other hand, the cellular monolayers of the invention are particularly advantageous in that they closely mimic the endogenous tissues in their behavior and functionalities.

In another aspect, the invention thus relates to a method for assessing the permeability of a compound, said method comprising the steps of:
i) growing adherent cells according to the method of invention until a mature cellular monolayer is obtained;
ii) injecting the test compound in the upper compartment 405 of the device of the invention;
iii) drawing the culture medium from lower compartment 105 of the device of the invention;
iv) measuring the quantity of test compound in said culture medium; and
v) estimating the permeability of said compound.

The quantity of test compound in step iv) may be measured by any method known to the person of skills in the art.

Control molecules with known permeability may be used in the method of the invention in order to classify said test compound as having high or low permeability. According to a particular embodiment of the method of the invention, a permeability control is added in step ii) and the permeability of this control is measured and compared to the permeability for the said test compound. Low and high permeability markers (i.e., controls) are known in the art. Examples of low or zero permeability markers include such molecules as e.g., mannitol, polyethylene glycol (PEG), dextran, inulin, and Lucifer yellow.

The flux of a low or zero permeability paracellular marker molecule (e.g., mannitol, polyethylene glycol (PEG), dextran, inulin, Lucifer yellow) may be used in each study to provide evidence for tissue or cell layer integrity. These markers demonstrate the presence of an intact physical barrier for drug transport.

Preferably, said low permeability marker is Lucifer yellow.

It will be appreciated that low permeability markers such as Lucifer yellow can also be used to detect any change in the permeability of a mature cellular monolayer of the invention. For example, if a compound were to increase the general permeability of said monolayer, this would lead to more low permeability marker (e.g. Lucifer yellow) being present in the culture medium in the lower compartments 105. Thus, in yet another aspect, the invention relates to a method for screening compounds for their capacity to alter the permeability properties of a mature cellular monolayer to a low permeability marker, particularly Lucifer Yellow; the said method comprising the steps of:
i) growing adherent cells according to the method of invention until a mature cellular monolayer is obtained;
ii) injecting the test compound in the upper compartments 405 of the device of the invention;
iii) injecting the low permeability marker in the upper compartments 405 of the device of the invention;
iv) drawing the culture medium from lower compartment 105 of the device of the invention;
v) measuring the quantity of said low permeability marker in said culture medium; and
vi) estimating the permeability for said low permeability marker.

In a preferred embodiment of the method of the invention, the permeability for said low permeability marker is also estimated by the method of the invention in the absence of the said test compound, and the two estimated permeability values are compared.

The examples that follow are merely exemplary of the scope of this invention and content of this disclosure. One skilled in the art can devise and construct numerous modifications to the examples listed below without departing from the scope of this invention.

### EXAMPLES

### Example 1

### Materials and Methods

### Cell culture conditions on conventional Transwell® culture inserts

The human brain microvessel endothelial cells hCMEC/D3 were grown on Transwell® culture inserts (0.4 mm pore size; Corning, Lowell, MA, USA) and coated with 150 µg/mL rat tail collagen type I (R&D Systems, Minneapolis, MN, USA). The seeding density was 40-50,000 cells/cm2. The medium contained Endothelial GROwing microvascular basal medium (EndoGROTM-MV) is supplemented with 0.2% EndoGRO- Low Serum Supplement, 5 ng/mL recombinant human epidermal growth factor (rh EGF), 10 mM L-Glutamine, 1µg/mL hydrocortisone hemisuccinate, 0.75U/mL heparin sulfate, 50µg/mL ascorbic acid and 5% fetal bovine serum (Millipore, EndoGROTM- MV complete media and supplement kit), 10mM Lithium chloride, 10 µM Resveratrol. Cells were maintained at 37°C for 6 days in a humidified air with 5% CO2. The medium was changed 3-4 days after seeding. Cellular growth was monitored every day by inspection using phase-contrast microscopy. All experiments were performed at day 6.

### Device 1

### * Experimental set up and mechanical modulation of hCMEC/D3 cell line

The semi porous membrane of device 1 was coated with 150µg/mL human collagen type IV and fibronectin 0.1% before hCMEC/D3 seeding at 5x105 cells. After 4 days of static cultures in the device 1, the medium was refreshed and hCMEC/D3 cells were exposed to shear stress at a flow rate of 12 mL/min (10 dynes/cm²) during 3 days (Redmond et al, In Vitro Cell Dev Biol Anim. 1995 Sep;31(8):601-9). Then, BBB integrity was validated through the following read out.

### * Validation of the set-up

To validate the developed dynamic device 1, the following read-out were used: -(i) cell imaging to characterize the mature cell monolayer, regarding the expression of BBB markers (for example: tight junction proteins, transporters, etc..), (ii) TEER measurement for assessing the tightness of the mature cell monolayer, (iii) permeability assays to assess the permeability of reference agents, such as Lucifer Yellow, and any test compound of interest

### Fluorescence Microscopy

HCMEC/D3 cells were cultivated on Transwell® culture insert as well as on device 1 during 6 or 7 days respectively. Cells were fixed with ice cold ethanol for 10 minutes at -20°C for VE-Cadherin, ZO-1, β-catenin, Occludin, Claudin-3, Claudin-5 protein staining or F-Actin. Cells were permeabilized with 0.1% Triton X-100 in PBS for 10 minutes at room temperature and blocked with 3% bovine serum albumin in PBS for 30 minutes. Cultures were incubated with the indicated primary antibodies in blocking solution overnight at 4°C. Cultures were rinsed with PBS and left in secondary antibodies for 1 hour protected from light (Jackson Immunoresearch Laboratories, West Grove, PA, USA). Labeled samples were washed with PBS and counter stained with DAPI, finally mounted in Glycergel medium (Dako Inc., Carpinteria, CA, USA). Fluorescence microscopy was performed with a Zeiss Axio Observer Z1 microscope, using a x40 Oil Objective. Acquisitions were made with the MetaMorph 7 software (Molecular Devices, Sunnyvale, CA, USA).

### Permeability assays

### * Transwell® culture inserts

HCMEC/D3 cells were grown on Transwell® culture insert during 6 days. Culture medium was removed and replaced by Hank's buffered salt solution (HBSS) transport buffer containing 10mM HEPES, and 1 mM sodium pyruvate (Life technologies, UK) in the lower chamber. In the upper chamber, the transport buffer was supplemented with 50 mM Lucifer Yellow (LY) salt (Sigma- Aldrich), as indicated. Incubations were performed in triplicates at 37°C, 95% humidity, and 5% CO2 as previously described (Weksler et al, 2005). Samples were analyzed using a fluorometer (Fusion, Packard Bioscience Company, Meriden, CT, USA). Results are expressed as Permeability coefficients Pe in 10-3cm/min.

### *Device 1

HCMEC/D3 cells were grown on device 1 during 7 days under static only or static and flow conditions. Culture medium was removed and replaced by Hank's buffered salt solution (HBSS) transport buffer containing 10mM HEPES, and 1 mM sodium pyruvate (Life technologies, UK) in the lower chamber. Equilibrate volume were determined and validated in order to have a strictly passive diffusion of the Lucifer Yellow. Incubations were performed in triplicate at 37°C, 95% humidity, and 5% CO2. Samples were analyzed using a fluorometer (Fusion, Packard Bioscience Company, Meriden, CT, USA). Results are expressed as Permeability coefficients Pe in 10-3cm/min.

### TEER measurements

To monitor hCMEC/D3 cell confluence and tightness integrity, TEER was measured at day 6 or 7 on Transwell® and device 1 respectively. For measurement of TEER, chopsticks electrodes were connected to EVOM device (WPI). To calculate TEER, initial background resistance of coated filter was subtracted from total resistance and normalized to cell surface area.

### Drug accumulation studies

The functional activity of P-gp and BCRP in hCMEC/D3 cells was performed using the Calcein-AM (1µM) and Bodipy Prazosin (1µM) substrates respectively. Cells cultivated in Endogro medium at confluence on Transwell® culture inserts (static) or device 1 (static and flow) were preincubated for 30min at 37°C with culture medium with or without the efflux transporter inhibitor Verapamil/P-gp (40µM) or KO-143/BCRP (10µM). Cellular uptake of the fluorescent substrates Calcein-acetoxymethylester (calcein-AM) and bodipy-prazosin was incubated for 1h. Cells were rapidly placed on ice, washed three times with ice-cold PBS and lysed with 5% of Triton during 20 min. The amount of fluorescent substrates retained by the cells was determined in a Mithras fluorescence plate reader with a 485-nm bandpass excitation/emission filter.

### In vitro In vivo correlation

Permeability assays for a large number of compounds with different physico-chemical properties were carried out in static (Transwell®) and flow (Device 1) conditions:, Prazosin hydrochloride, Quinidine, , Verapamil, at a final concentration of 10µM. The procedure of permeability assays was the same as for Lucifer yellow described above for both systems.

### Statistical Analysis

Data are presented as means ± s.d. Statistical analysis was performed using Student's t tests with differences between means considered significant when p<0.05.

### Results

### Design of the Device 1 for cell culture under laminar flow and permeability assays

The device 1 is composed of two compartments separated by a semi-porous membrane (0.4 µm pores; total area =xx) on which cells are grown. The upper compartment can be submitted to laminar shear stress (1-20 dynes/cm2, so-called "flow compartment") and is designed to allow for a regular shear stress all over the cell monolayer. The lower compartment is divided in 6 wells, maintained full of medium to allow nutrients access to the cells through the semi-porous membrane; each well is accessible from the top of the device through a silicone channel.

After endothelial cells have been submitted to dynamic culture conditions in this device for several days (as indicated below), the upper flow compartment is replaced by a "permeability assay compartment", composed of 6 wells fitting with the 6 wells of the lower compartment. In this configuration, the device 1 is compatible with permeability assays performed in static condition.

This device was used here to submit to shear stress (laminar flow?) the hCMEC/D3 cell line, a validated model of human brain endothelium, in order to obtain a mature cellular monolayer with a blood-brain barrier phenotype.

### Laminar flows promotes hCMEC/D3 cell quiescence.

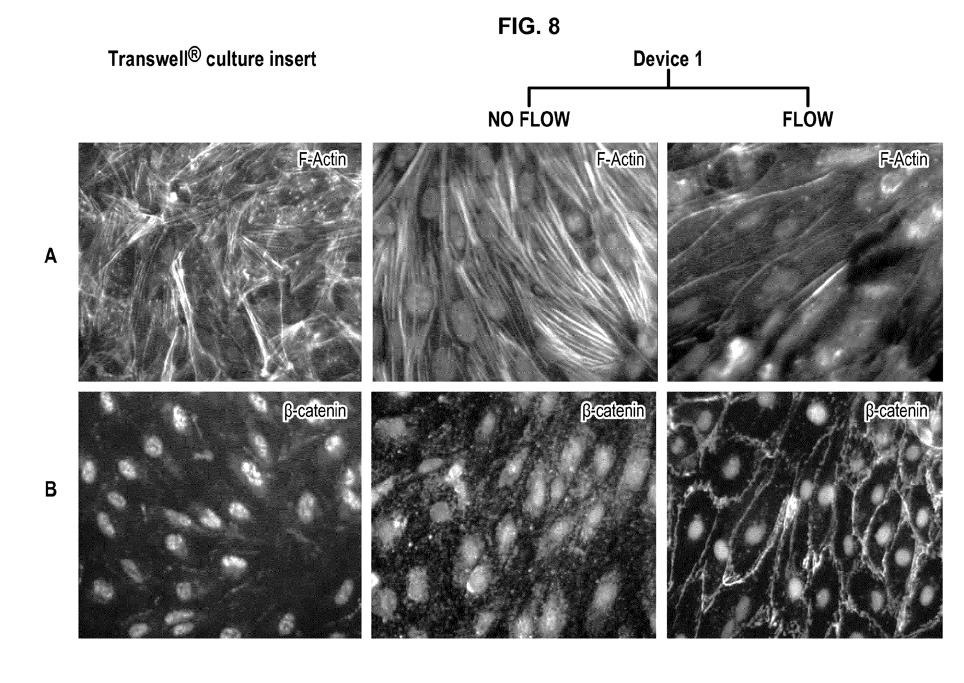

HCMEC/D3 cells were grown on the semi-porous membrane of the device 1 for 4 days in static condition, until confluence, then submitted for 3 days to a laminar flow of 10 dynes/cm2 (or maintained in static condition for the same period of time, as control). Cells were then stained for F-actin cytoskeleton using fluorescent Phalloidin, and for proliferation / quiescence markers, using betacatenin antibodies; hCMEC/D3 cells grown to confluence on Transwell® devices for 6 days (as previously described) were used as reference. As shown in Fig.8, Phalloidin staining revealed numerous actin stress fibers in hCMEC/D3 cells on Transwell® devices as well as in the device 1 in static condition. By contrast, hCMEC/D3 cells submitted to laminar flow (10 dynes/cm2) in the device 1 exhibited much less actin stress fibers, F-actin being mostly localized at the cell cortex: this pattern was highly reminiscent of that previously described in vascular endothelium in situ.

In addition, when hCMEC/D3 cells were stained with β-catenin antibodies, a dual localization of β-catenin was observed at cell-cell junctions and in the nucleus, with cells grown on the Transwell® devices or in the device 1 in static condition, in agreement with the known dual activity of β-catenin as a VE-cadherin partner and as a nuclear transcription factor in activated cells. By contrast, hCMEC/D3 cells submitted to laminar flow (10 dynes/cm2) in the device 1 displayed no significant nuclear localization, strongly suggesting that hCMEC/D3 cells under flow were mostly quiescent cells. Taken together, these results indicate that laminar flow strengthens the F-actin binding junctional complexes in hCMEC/D3 cells and maintains them in a quiescent state, closer to the *in situ* properties of vascular endothelium.

### Flow ameliorates expression of adherens and tight junction proteins.

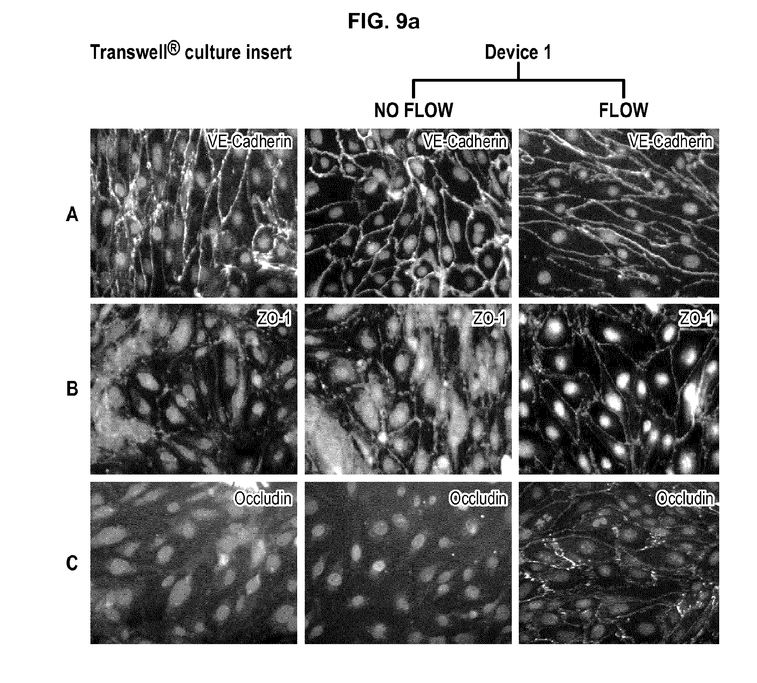

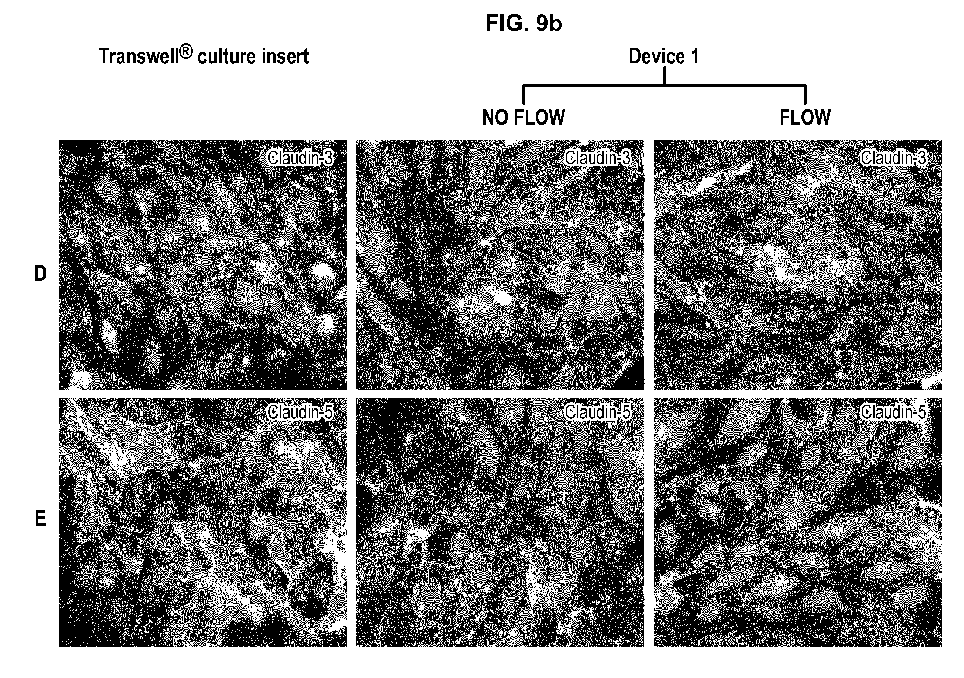

In this second series of experiments, we assessed the role of laminar shear stress in the expression and maturation of adherens and tight junction (AJ & TJ) proteins. Regarding junctional complexes, hCMEC/D3 cells cultivated on Transwell® culture inserts or on device 1 in static condition, express ZO-1, Occludin, Claudin-3, Claudin-5 as well as VE-Cadherin , markers of tight junctions (TJ) and adherens junction (AJ) complexes, respectively, and are properly localized at cell-cell junctions (Figure 9A-E).

The impact of a pulsatile laminar flow on hCMEC/D3 cell line changes the pattern of expression of AJ and TJ complexes. VE-Cadherin staining seems more mature, thinner; cells are more elongated compared to static condition on device 1, suggesting a reorganization of the monolayer under flow. ZO-1 staining suggests a migration of the protein from the nucleus to the plasma membrane. Occludin staining seems more membranous in spite of perinuclear and cytoplasmic staining under flow. Finally, no difference is observed concerning Claudin-3 expression under static or flow condition; Claudin-3 is expressed continuously at cell-cell junctions. In contrast, we clearly observe an increased expression of Claudin-5, a key tight junction involved in the permeability of the BBB, under flow compared to static condition on Device 1 set up. A nuclear staining is also present. Those modification were not necessary accompanied by a cell orientation to flow. Altogether these results tend closely to in vivo like immunostaining.

### Laminar flow increases ABC-Transporter activity in hCMEC/D3 cells

In conjunction with the presence of TJs which limit the passive diffusion of hydrophilic molecules from the blood to the brain, the BBB phenotype results from the expression of several multidrug resistance proteins of the ATP binding cassette (ABC) transporter family which largely contribute to the strict control of the permeability to small hydrophobic compounds. In order to assess a putative role of laminar flow in regulating these transporters, we measured the activity of BCRP and P-gp, the two major ABC-transporters at the human BBB, in hCMEC/D3 cells in static or dynamic culture conditions. The activity of.BCRP was assessed in each condition by quantification of the cellular content of its fluorescent substrate Bodipy-Prasozin in the presence or absence of the BCRP inhibitor KO143. Similarly, we measured P-gp activity by measuring the cellular content of green-fluorescent calcein, a P-gp substrate formed by intracellular hydrolysis of calcein AcetoxyMethyl ester (calcein-AM), a non fluorescent cell-permeant dye, in the presence or absence of the P-gp inhibitor verapamil.

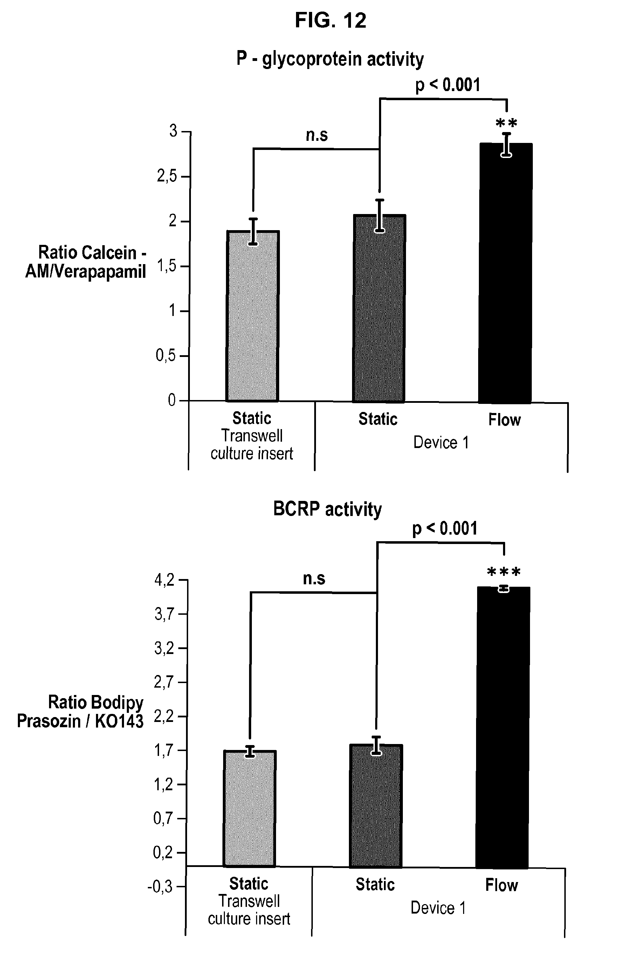

As presented in Fig. 12, the KO143-dependent accumulation of Bodipy-prazosin in hCMEC/D3 cells was strongly enhanced under shear stress, as compared with the static conditions in Transwell® inserts or in the device 1, revealing a sharp increase in BCRP activity under flow. Similarly, the P-gp activity was clearly stimulated by laminar flow, although to a lower extent than BCRP, as shown by the increased verapamil- dependent accumulation of calcein in hCMEC/D3 cells

The data indicate that, in addition to controlling TJ integrity in hCMEC/D3 cells, laminar flow in the device 1 also increases BCRP and P-gp activities: they likely reflect a further differentiation of hCMEC/D3 cells towards a BBB phenotype.

### Permeability of hCMEC/D3 cells under flow to a series of drugs or drug candidates

To evaluate further the permeability characteristics of hCMEC/D3 cell line, we carried out permeability assays with several standard compounds with different physico-chemical properties: Prazosin hydrochloride, Quinidine, and Verapamil.

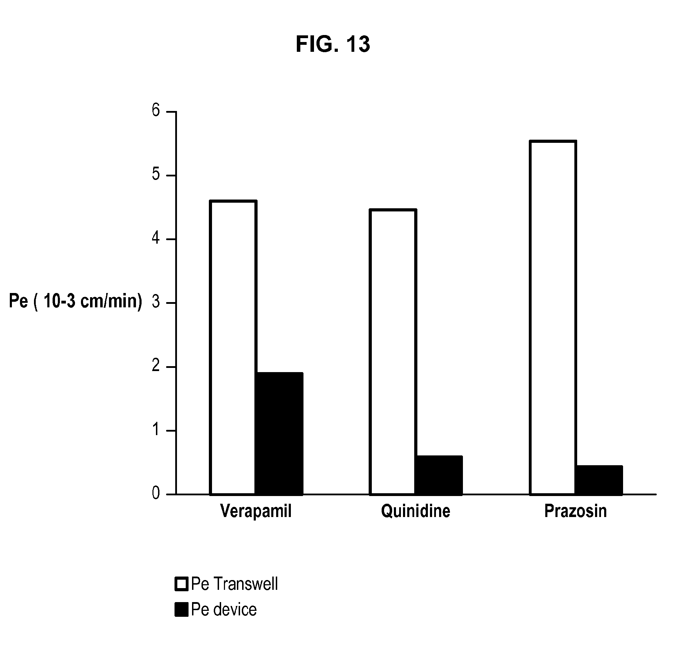

As shown in figure 13, permeability of diazepam, a passive diffusion cell marker, is increased under flow condition compared to static. Interestingly, permeability data for prazosin hydrochloride, quinidine and verapamil, key efflux drug transporters of P-gp and BCRP, are significantly decreased under flow condition which confirms that shear stress positively impact on the activity and expression of key BBB markers and tend to an in vivo BBB like phenotype.

Permeability coefficients (Pe in 10-3 cm.min-1) calculated with hCMEC/D3 cells grown to confluence on Transwell® culture inserts and under flow within device 1 system were correlated with in vivo permeability data (expressed as Kin transport coefficient values) obtained by brain perfusion of adult rats or mice (reference), which are considered quantitatively similar to human values (Table1).

**Table 1**

| | Pe Transwell® | Pe Device 1 | Kin value |
|---|---|---|---|
| Verapamil | 4.6 | 1.9 | 2.04 |
| Quinidine | 4.46 | 0.59 | 0.6 |
| Prazosin hydrochloride | 5.54 | 0.44 | 0.96 |

### Conclusion

The adherens and tight junctions of the BBB are key elements to be considered for the restrictive paracellular permeability observed under flow condition. Junctional expression of certain claudins and occludin as well as continuous junctional expression of ZO-1, all demonstrated in hCMEC/D3 cells, correlate in situ with functional tight junctions. These results support the conclusion that hCMEC/D3 cells further maintain the overall adherens and tight junction organization known to be present in brain endothelium.

To date, the device 1 constitutes a unique mean for phenotypic optimization of the hCMEC/D3 cell line, which triggers in dynamic culture most of the unique structural and biochemical properties of brain endothelium in vivo.

We clearly demonstrate that the activity of P-gp and BCRP transporters is significantly increased under flow condition.

To sum up, the pharmaceutical industry needs simple, reliable, in vitro BBB models for predicting the brain penetrability of CNS drugs. A model to serve as a permeability screen should display a physiologically realistic cell architecture, a restrictive paracellular pathway, and functional expression of transporter mechanisms and ease of culture to facilitate drug screening. For the first time in our knowledge we report this new innovative dynamic set up which answer positively to all this issues.

This set up will in fact open a new way for the study of other barriers (e.g HUVEC, HDMEC ...) for ADME and toxicology study and is presented as an additional option of Caco-2 model for high throughput screening.

### Example 2

Circulating endothelial progenitor (CEP) cells were injected directly on the semi-porous membrane of device 1 and addressed for static culture during 4 days in presence of EGM2 medium and 10ng/µL of VEGF. After 4 days of culture, CEP cells were placed under dynamic condition at 16 dynes/cm² associated with a high concentration of VEGF at 50ng/µL for 3 days. At day 7, CEP cells status under static or dynamic condition were analyzed by immunofluorescence and qPCR approaches.

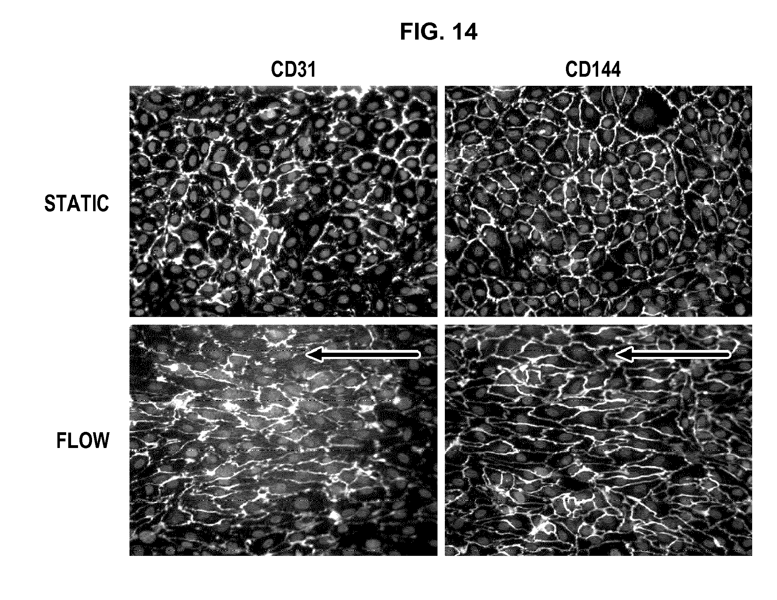

By immunofluorescence approach, CEP cells under static and flow condition were stained by two regular endothelial markers: CD31 and CD144 (Figure 14). Interestingly, CEP cells demonstrated under flow condition a reorientation under flow condition compared to static.

These results, highlighted by CD31 and CD144 immunostaining, demonstrate a positive answer of CEP cells to shear stress with a clear reorganization in the same direction of flow.

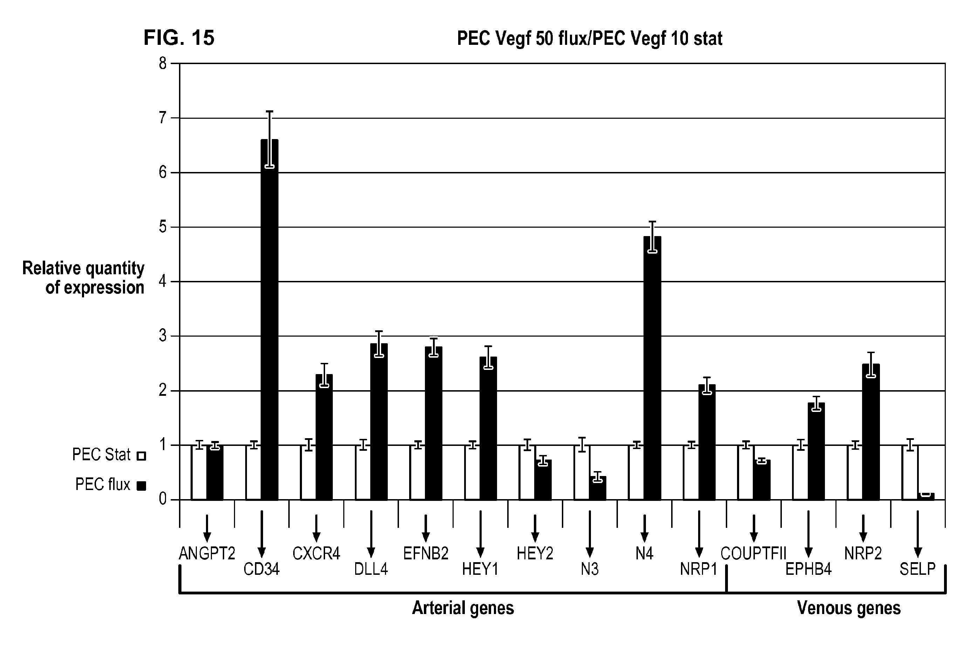

By qPCR approach, CEP cells cultivated under static and flow conditions associated with a high concentration of VEGF were characterized by screening 10 keys markers specific to arterial phenotype. Over those 10 key arterial markers, 7 of them are specifically up-regulated under flow condition compared to static: CD34, CXCR4, DLL4, EFNB2, HEY1, N4 and NRP1 (Figure 15).

These results clearly suggest an arterialization of CEP cells thanks to shear stress and associated with a high concentration of VEGF.

## Claims

1. Device for cultivating cells comprising:
- a lower part comprising at least two lower compartments, a lower wall separating the two lower compartments, and a semi-porous membrane covering the lower compartments, the semi-porous membrane comprising an upper surface intended to receive the cells;
- a first upper part and a second upper part intended to be fitted onto the lower part, in order to obtain selectively:
- a first configuration of the device wherein the first upper part is fitted onto the lower part, the first upper part forming an upper compartment wherein a culture medium can flow, in order to apply shear stress to the cells;
- a second configuration of the device wherein the second upper part is fitted onto the lower part, the second upper part comprising at least two upper compartments, a wall separating the two upper compartments, and injection openings for injecting a substance to be tested in each upper compartment, the wall of the second upper part facing the wall of the lower element and being in contact with the semi-porous membrane so as to prevent a leakage of the injected substance from an upper compartment to another.

2. Device according to claim 1, wherein each upper part comprises upper channels and the lower part comprises lower channels leading to the lower compartments, the upper channels being arranged so that when the upper part is fitted onto the lower part, each upper channel is connected to a lower channel.

3. Device according to claim 2, wherein the upper channels and the lower channels are arranged such that each lower compartment communicates with at least two upper channels via the lower channels.

4. Device according to claim 2 or 3, wherein at least some of the upper channels are filled with silicon stoppers.

5. Device according to any claims 1 to 4, wherein the lower part and the upper parts are made of a transparent material.

6. Device according to any claims 1 to 5, wherein the wall separating the lower compartments is thicker than the facing wall separating the upper compartments of the second upper element.

7. Use of a device according to any of the preceding claims for cultivating cells.

8. Use according to claim 7, wherein cells are injected in the lower compartments.

9. Use according to any claims 7 or 8, wherein at least one electrode is inserted in at least one lower compartment.

10. A method for cultivating adherent cells, said method comprising the following steps:
i) injecting culture medium in the lower compartment of a device according to any of the preceding claims;
ii) seeding said device with adherent cells;
iii) growing said cells in static conditions until a cellular monolayer is formed; and
iv) applying to said cellular monolayer a shear stress comprised between 0.01 dyne/cm² to 30 dynes/cm², until a mature cellular monolayer is obtained, preferably during 2 to 4 days, more preferably 3 days.

11. The method according to claim 10, wherein said adherent cells are selected in the group of endothelial cells and epithelial cells, in particular ependymal cells.

12. Cells obtained by the method according to any one of claims 10 and 11.

13. A method for assessing the permeability of a compound, said method comprising the steps of:
i) growing adherent cells according to the method of any one of claims 10 or 11;
ii) injecting the test compound in the upper compartment of the device according to any one of claims 1 to 9;
iii) drawing the culture medium from lower compartment of the said device;
iv) measuring the quantity of test compound in said culture medium; and
v) estimating the permeability of said compound.

14. A method for screening compounds for their capacity to alter the permeability properties of a mature cellular monolayer to a low permeability marker, particularly Lucifer Yellow; the said method comprising the steps of:
i) growing adherent cells according to the method of any one of claims 10 or 11;
ii) injecting the test compound in the upper compartments of the device according to any one of claims 1 to 9;
iii) injecting the low permeability marker in the upper compartments of the said device;
iv) drawing the culture medium from lower compartment of the said device;
v) measuring the quantity of said low permeability marker in said culture medium; and
vi) estimating the permeability for said low permeability marker.
